(19) Europäisches Patentamt
European Patent Office
Office européen des brevets

(11) Veröffentlichungsnummer: **0 175 188**
**A1**

(12) **EUROPÄISCHE PATENTANMELDUNG**

(21) Anmeldenummer: **85110918.1**

(22) Anmeldetag: **30.08.85**

(51) Int. Cl.⁴: **C 07 D 233/61**, C 07 C 43/225, C 07 C 149/34, C 07 C 149/42, C 07 C 149/437, A 01 N 47/38

(30) Priorität: **11.09.84 JP 188778/84**
**25.02.85 JP 34642/85**

(43) Veröffentlichungstag der Anmeldung: **26.03.86**
**Patentblatt 86/13**

(84) Benannte Vertragsstaaten: **AT BE CH DE FR GB IT LI NL SE**

(71) Anmelder: **NIHON TOKUSHU NOYAKU SEIZO K.K., No.4, 2-chome, Nihonbashi Honcho Chuo-ku, Tokyo 103 (JP)**

(72) Erfinder: **Kume, Toyohiko, 6-7-8, Asahigaoka, Hino-shi Tokyo (JP)**
Erfinder: **Kurahashi, Yoshio, 47-15, Oya-machi, Hachioji-shi Tokyo (JP)**
Erfinder: **Isono, Kunihiro, 2-32-4, Asahigaoka, Hino-shi Tokyo (JP)**
Erfinder: **Sakawa, Shinji, 1-14-13, Tamadaira, Hino-shi Tokyo (JP)**
Erfinder: **Matsumoto, Noboru, 39-15, Namiki-cho, Hachioji-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

(54) **Carbamoylimidazol-Derivate.**

(57) Die vorliegende Erfindung betrifft neue Carbamoylimidazol-Derivate, Verfahren zu ihrer Herstellung und ihre Verwendung als Fungizide für Landwirtschaft und Gartenbau.

Die Verbindungen der allgemeinen Formel

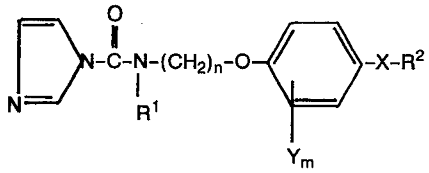

(I)

in der $R^1$, $R^2$, X, Y, m und n die in der Beschreibung angegebenen Bedeutungen haben, können durch Reaktion der entsprechenden Aminoalkyl-(fluoroalkoxyphenyl)- oder -(fluoroalkylthiophenyl)ether und Carbonyl-di-imidazol hergestellt werden.

Die neuen Carbamoylimidazol-Derivate zeigen hervorragende Wirksamkeit gegen phytopathogene Pilze auf dem Gebiet der Landwirtschaft und des Gartenbaus.

EP 0 175 188 A1

# Carbamoylimidazol-Derivate

Die vorliegende Erfindung betrifft neue Carbamoylimidazol-Derivate, Verfahren zu ihrer Herstellung und ihre Verwendung als Fungizide für Landwirtschaft und Gartenbau.

Es wurde bereits offenbart, daß bestimmte Carbamoylimidazol-Derivate fungizide Aktivität besitzen (vgl. die JP-OS 31047/1975 bzw. die GB-Patentanmeldung 1 469 772).

Nunmehr wurden neue Carbamoylimidazol-Derivate der Formel (I)

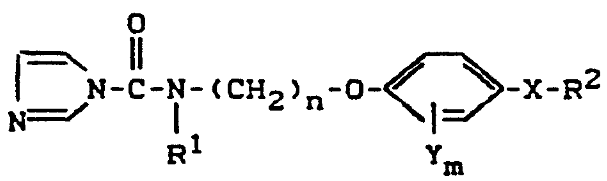

(I)

gefunden, in der

$R^1$ eine Niederalkyl-Gruppe, eine Niederalkoxy-niederalkyl-Gruppe oder eine Cycloalkyl-Gruppe mit 3 bis 8 Kohlenstoff-Atomen bezeichnet,

$R^2$ eine fluoro-substituierte Niederalkyl-Gruppe bezeichnet,

X Sauerstoff oder Schwefel bezeichnet,
Y Halogen oder Niederalkyl bezeichnet,
m die Zahlen 0, 1 oder 2 bezeichnet und
n die Zahlen 2, 3, 4, 5 oder 6 bezeichnet.

Carbamoylimidazol-Derivate der Formel (I) werden erhalten, wenn

(a) die Verbindungen der Formel (II)

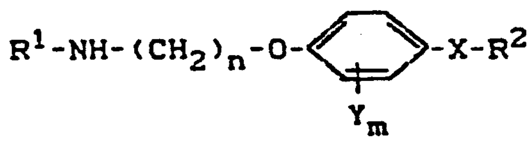 (II) ,

in der $R^1$, $R^2$, X, Y, m und n die im Vorstehenden angegebenen Bedeutungen haben, mit N,N'-Carbonyldiimidazol der Formel

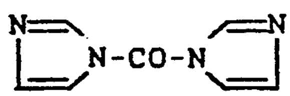 (III) ,

gegebenenfalls in Gegenwart von Säure-Acceptoren und inerten Lösungsmitteln, umgesetzt werden oder

(b) die Verbindungen der Formel (IV)

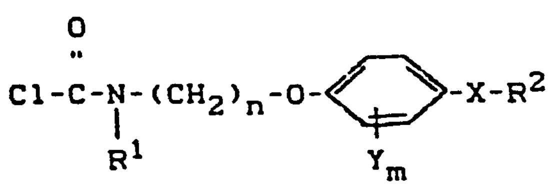 (IV) ,

in der $R^1$, $R^2$, X, Y, m und n die im Vorstehenden angegebenen Bedeutungen haben, mit der Imidazol-Verbindung der Formel (V)

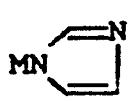 (V) ,

in der M ein Wasserstoff-Atom oder ein Alkalimetall-Atom bezeichnet, gegebenenfalls in Gegenwart von Säure-Acceptoren und inerten Lösungsmitteln umgesetzt werden.

Die neuen Carbamoylimidazol-Derivate der Formel (I) zeigen stark ausgeprägte fungizide Eigenschaften beim Einsatz in Landwirtschaft und Gartenbau.

Überraschenderweise weisen die Verbindungen gemäß der vorliegenden Erfindung eine wesentlich größere fungizide Wirkung in Landwirtschaft und Gartenbau als die aus dem vorgenannten Stand der Technik bekannten Verbindungen auf.

Obwohl der gattungsgemäße Rahmen des vorgenannten Standes der Technik die Verbindungen der Formel (I) gemäß der vorliegenden Erfindung abstrakt begrifflich einschließt, fehlen in der Beschreibung des Standes der Technik jegliche Einzelangaben, die eine spezielle Beschreibung der vorliegenden Erfindung geben oder diese auch nur nahelegen könnten.

Unter den Carbamoylimidazol-Derivaten der Formel (I) sind bevorzugte Verbindungen diejenigen, in denen

$R^1$ eine Alkyl-Gruppe mit 1 bis 4 Kohlenstoff-Atomen, eine Alkoxy-alkyl-Gruppe mit insgesamt 2 bis 4 Kohlenstoff-Atomen oder eine Cycloalkyl-Gruppe mit 5 bis 6 Kohlenstoff-Atomen bezeichnet,

$R^2$ eine Fluoroalkyl-Gruppe mit 1 bis 4 Kohlenstoff-Atomen und 1 bis 3 Fluor-Atomen bezeichnet,

Y Chlor oder Methyl bezeichnet und

X, m und n die im Vorstehenden angegebenen Bedeutungen haben.

Besonders bevorzugt sind diejenigen Verbindungen der Formel (I), in denen

$R^1$ eine Alkyl-Gruppe mit 1 bis 4 Kohlenstoff-Atomen, eine Alkoxy-alkyl-Gruppe mit insgesamt 2 bis 4 Kohlenstoff-Atomen oder Cyclopentyl bezeichnet,

$R^2$ trifluoro-substituiertes Alkyl mit 1 bis 4 Kohlenstoff-Atomen bezeichnet,

X Sauerstoff oder Schwefel bezeichnet,

Y Chlor oder Methyl bezeichnet,

m die Zahlen 0, 1 oder 2 bezeichnet und

n die Zahlen 2, 3, oder 4 bezeichnet.

Ganz besonders bevorzugte Carbamoylimidazol-Derivate der Formel (I) sind diejenigen Verbindungen, in denen

$R^1$ eine Alkyl-Gruppe mit 3 bis 4 Kohlenstoff-Atomen oder Ethoxyethyl bezeichnet,

$R^2$ Trifluoromethyl oder 2,2,2-Trifluoroethyl bezeichnet,

X Sauerstoff oder Schwefel bezeichnet,

Y Chlor bezeichnet,

m die Zahlen 0, 1 oder 2 bezeichnet und

n die Zahl 2 bezeichnet.

Speziell erwähnt seien die folgenden Verbindungen:

1-[N-2-[2,6-Dichloro-4-(2,2,2-trifluoroethylthio)-phenoxy]ethyl-N-propyl-carbamoyl]imidazol,

1-[N-2-[2,6-Dichloro-4-(2,2,2-trifluoroethylthio)-phenoxy]ethyl-N-sec-butyl-carbamoyl]imidazol,

1-[N-2-(4-Trifluoromethylthiophenoxy)ethyl-N-sec-butyl-carbamoyl]imidazol,

1-[N-2-[2,6-Dichloro-4-(trifluoromethylthio)-phenoxy]ethyl-N-propyl-carbamoyl]imidazol,

1-[N-3-(4-Trifluoromethylthiophenoxy)propyl-N-sec-butyl-carbamoyl]imidazol,

1-[N-5-(4-Trifluoromethylthiophenoxy)pentyl-N-propyl-carbamoyl]imidazol,
1-[N-2-(4-Trifluoromethoxyphenoxy)ethyl-N-propyl-carbamoyl]imidazol,
1-[N-2-(2,6-Dichloro-4-trifluoromethoxyphenoxy)ethyl-N-propyl-carbamoyl]imidazol und
1-[N-3-(4-Trifluoromethoxyphenoxy)propyl-N-sec-butyl-carbamoyl]imidazol.

Wenn beispielsweise N-[2-[2,6-Dichloro-4-(2,2,2-trifluoroethylthio)-phenoxy]ethyl]-N-propylamin und N,N'-Carbonyldiimidazol als Ausgangsmaterialien eingesetzt werden, läßt sich der Ablauf der Reaktionsvariante (a) durch die folgende Gleichung darstellen:

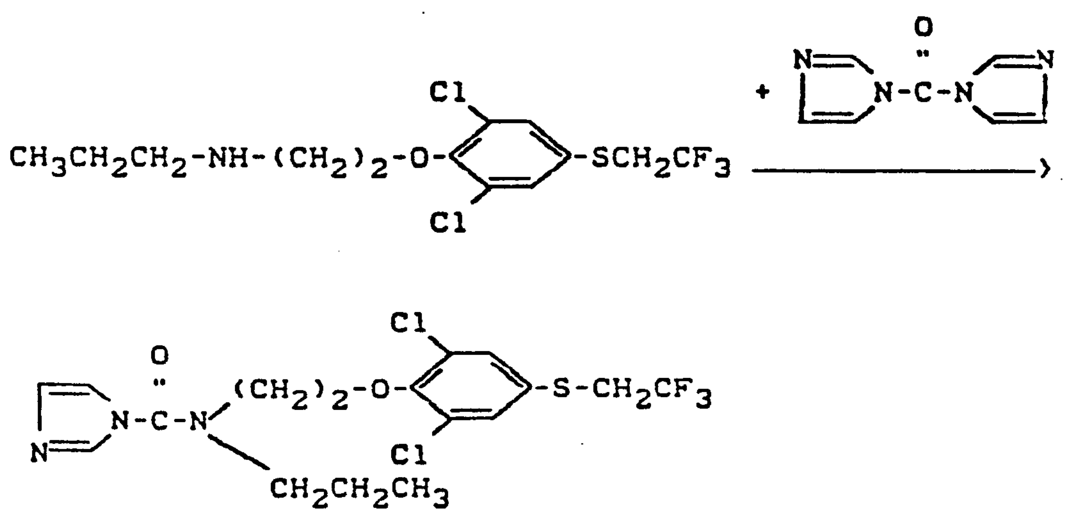

Wenn beispielsweise N-(sec-Butyl)-N-[(4-trifluoromethoxy)phenoxyethyl]amidoameisensäurechlorid und Imidazol als Ausgangsmaterialien eingesetzt werden, läßt sich der Ablauf der Reaktionsvariante (b) durch die folgende Gleichung darstellen:

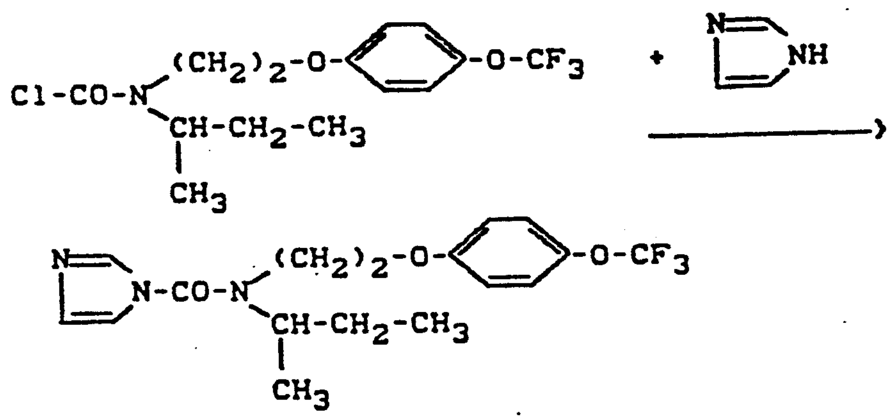

Als Ausgangsstoffe in der Reaktionsvariante (a) gemäß der vorliegenden Erfindung werden die Verbindungen der allgemeinen Formel (II) benötigt. In dieser Formel haben $R^1$, $R^2$, X, Y, m und n vorzugsweise die bereits im Vorstehenden in Verbindung mit den bevorzugten Bedeutungen der Substituenten in der allgemeinen Formel (I) angegebenen Bedeutungen.

Die Verbindungen der allgemeinen Formel (II) sind neu und werden durch Umsetzung von Verbindungen der Formel (VI)

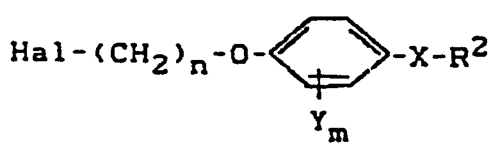

(VI)

,

in der

$R^2$, X, Y, m und n die im Vorstehenden angegebenen Bedeutungen haben und

Hal ein Halogen-Atom bezeichnet,

mit Amino-Verbindungen der Formel (VII)

$$R^1\text{-}NH_2 \qquad (VII),$$

in der
$R^1$ die im Vorstehenden angegebene Bedeutung hat,
erhalten.

Die Zwischenprodukte der allgemeinen Formel (VI) sind ebenfalls neu. Sie werden erhalten, wenn Verbindungen der Formel (VIII)

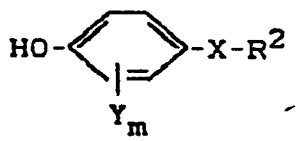

(VIII) ,

in der
$R^2$, X, Y, und m die im Vorstehenden angegebenen Bedeutungen haben,
mit Dihalogeno-Verbindungen der Formel (IX)

$$Hal\text{-}(CH_2)_n\text{-}Hal \qquad (IX),$$

in der
n und Hal die im Vorstehenden angegebenen Bedeutungen haben,
gegebenenfalls in Gegenwart von Säure-Acceptoren und inerten Lösungsmitteln, umgesetzt werden.

Als Beispiele für die neuen Zwischenprodukte der Formel (VI) seien die folgenden Verbindungen erwähnt:
2-[4-(2,2,2-Trifluoroethylthio)-phenoxy]ethylbromid,
2-[4-(2,2,2-Trifluoroethylthio)-2-tolyloxy]ethylbromid,
2-[4-(2,2,2-Trifluoroethylthio)-3,5-xylyloxy]ethylbromid,
2-[2-Chloro-4-(2,2,2-trifluoroethylthio)-phenoxy]ethylbromid,

2-/¯2,6-Dichloro-4-(2,2,2-trifluoroethylthio)-phenoxy_7ethylbromid,
3-/¯4-(2,2,2-Trifluoroethylthio)-phenoxy_7propylbromid,
3-/¯2,6-Dichloro-4-(2,2,2-trifluoroethylthio)-phenoxy_7propylbromid,
4-/¯4-(2,2,2-Trifluoroethylthio)-phenoxy_7butylbromid,
4-/¯4-(2,2,2-Trifluoroethylthio)-2-tolyloxy_7butylbromid,
4-/¯4-(2,2,2-Trifluoroethylthio)-3,5-xylyloxy_7butylbromid,
4-/¯2-Chloro-4-(2,2,2-trifluoroethylthio)-phenoxy_7-butylbromid,
2-(4-Trifluoromethylthiophenoxy)ethylbromid,
2-(2-Chloro-4-trifluoromethylthiophenoxy)ethylbromid,
2-(2,6-Dichloro-4-trifluoromethylthiophenoxy)ethylbromid,
3-(4-Trifluoromethylthiophenoxy)propylbromid,
3-(2-Chloro-4-trifluoromethylthiophenoxy)propylbromid,
3-(2,6-Dichloro-4-trifluoromethylthiophenoxy)propylbromid,
4-(4-Trifluoromethylthiophenoxy)butylbromid,
4-(2-Chloro-4-trifluoromethylthiophenoxy)butylbromid,
2-(4-Trifluoromethoxyphenoxy)ethylbromid,
2-(2-Chloro-4-trifluoromethoxyphenoxy)ethylbromid,
2-(2,6-Dichloro-4-trifluoromethoxyphenoxy)ethylbromid,
3-(4-Trifluoromethoxyphenoxy)propylbromid,
3-(2-Chloro-4-trifluoromethoxyphenoxy)propylbromid und
3-(2,6-Dichloro-4-trifluoromethoxyphenoxy)propylbromid.

Die Verbindungen der Formel (VIII) sind zum Teil bereits bekannte Verbindungen (vgl. die JP-OSen 222094/1983 und 151151/1977). Als Beispiele seien erwähnt:

4-(2,2,2-Trifluoroethylthio)phenol,
4-(2,2,2-Trifluoroethylthio)-2-methyl-phenol,
4-(2,2,2-Trifluoroethylthio)-3,5-dimethyl-phenol,
2-Chloro-4-(2,2,2-trifluoroethylthio)phenol,
2,6-Dichloro-4-(2,2,2-trifluoroethylthio)phenol,
4-Trifluoromethylthiophenol,
2-Chloro-4-trifluoromethylthiophenol,
2,6-Dichloro-4-trifluoromethylthiophenol,
4-Trifluoromethoxyphenol,
2-Chloro-4-trifluoromethoxyphenol und
2,6-Dichloro-4-trifluoromethoxyphenol.

Einige dieser Verbindungen sind neu. Beispielsweise ist die Verbindung 2,6-Dichloro-4-(2,2,2-trifluoroethylthio)phenol neu und kann unter Einsatz des wohlbekannten 2,6-Dichlorophenols (vgl. Beilstein, Hauptwerk H 6, Seite 190) als Ausgangsstoff hergestellt werden, das mit Chlorsulfonsäure umgesetzt wird; das erhaltene 2,6-Dichloro-4-chlorosulfonylphenol wird mit Zink und Schwefelsäure reduziert, und das dadurch erhaltene 2,6-Dichloro-4-mercaptophenol wird anschließend mit Phenylsulfonsäure-(2,2,2-trifluoroethyl)ester in Gegenwart von Kaliumcarbonat in Dimethylformamid umgesetzt, wodurch die neue Verbindung der Formel

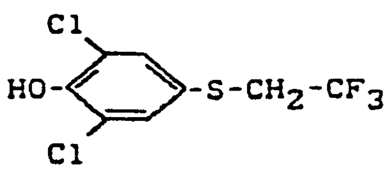

erhalten wird.

Die oben genannten Verfahren sind bereits im allgemeinen bekannt (vgl. beispielsweise J. Org. Chem. 31,

2672-74 (1966) und Organic Syntheses, Coll. Vol. 1, Seiten 504-506).

2,6-Dichloro-4-trifluoromethylthiophenol ist ebenfalls neu und kann unter Verwendung von trifluoroiodomethan an Stelle des 2,2,2-Trifluoroethylbenzolsulfonats gemäß der vorstehend beschriebenen Reaktion hergestellt werden.

2,6-Dichloro-4-trifluoromethoxyphenol ist ebenfalls neu, und der Reaktionsweg zu seiner Herstellung läßt sich wie folgt erläutern: Das bereits bekannte Trifluoromethoxyphenol (vgl. J. Org. Chem. 44, 2907-10 (1979)) wird in Kohlenstofftetrachlorid chloriert, was 2-Chloro-4-(trifluoromethoxy)phenol (bei etwa 20°C bis 30°C) und 2,6-Dichloro-4-(trifluoromethoxy)phenol (beim Siedepunkt der Reaktionsmischung) liefert.

Die Dihalogeno-Verbindungen der allgemeinen Formel (IX) sind wohlbekannt, und als Beispiele seien erwähnt:
Ethylenbromid,
Propylenbromid und
Butylenbromid.

Die Formel (VII) liefert eine allgemeine Definition für die als Primärprodukte in der Reaktionsvariante (a) benötigten Amino-Verbindungen. In dieser Formel hat $R^1$ vorzugsweise die bereits im Vorstehenden angegebenen Bedeutungen. Als Beispiele für die wohlbekannten Verbindungen der Formel (VII) seien erwähnt:
Methylamin,
Ethylamin,
n-Propylamin,

iso-Propylamin,
n-Butylamin,
sec-Butylamin,
2-Ethoxyethylamin,
Cyclopentylamin und
Cyclohexylamin.

Die im Vorstehenden im Zusammenhang mit der allgemeinen Reaktionsvariante (a) erläuterten Verbindungen der Formel (II) sind ebenfalls Ausgangsstoffe, die für die Herstellung der Verbindungen der allgemeinen Formel (IV) benötigt werden. Die letztgenannten Verbindungen, die die Ausgangsstoffe in der Reaktionsvariante (b) sind, werden durch Umsetzung der neuen Verbindungen der allgemeinen Formel (II) mit Phosgen oder mit Diphosgen (d.h. Trichloromethyl-chlorameisensäureester), vorzugsweise in einem Lösungsmittel wie Benzol oder Toluol bei Temperaturen zwischen 40°C und 120°C, erhalten (hierzu wird auch auf die Herstellungsbeispiele verwiesen).

Die Imidazolyl-Verbindungen der Formel (V) werden ebenfalls als Ausgangsstoffe in der Reaktionsvariante (b) gemäß der vorliegenden Erfindung gebraucht.

In dieser Formel bezeichnet M vorzugsweise Wasserstoff (d.h. Imidazol selbst wird bevorzugt), Natrium und Kalium.

Das Verfahren zur Herstellung der Verbindungen der allgemeinen Formel (I) gemäß der Verfahrensvariante (a) kann zweckmäßigerweise in einem Lösungsmittel oder Verdünnungsmittel durchgeführt werden. Geeignete Lösungsmittel sind sämtliche inerten Lösungsmittel. Zu diesen

zählen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Ethylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Reaktionsvariante (a) wird vorzugsweise in Gegenwart eines Säure-Acceptors durchgeführt. Beispiele für solche Säure-Acceptoren umfassen die Hydroxide, Carbonate, Hydrogencarbonate und Alkoholate von Alkalimetallen und tertiäre Amine wie Triethylamin, Diethylanilin und Pyridin.

Die Reaktionsvariante (a) kann innerhalb eines breiten Temperaturbereichs durchgeführt werden. Im allgemeinen wird sie bei einer Temperatur zwischen etwa -20°C und dem Siedepunkt der Reaktionsmischung, vorzugsweise zwischen etwa 0°C und etwa 100°C, durchgeführt. Die Reaktion wird vorzugsweise unter normalem atmosphärischem Druck durchgeführt, jedoch ist es auch möglich, unter erhöhtem oder vermindertem Druck zu arbeiten.

Die praktische Durchführung der Reaktionsvariante (a) gemäß der vorliegenden Erfindung kann so erfolgen, daß

die Zwischenprodukte der Formel (II) nicht isoliert werden. Alternativ ist es auch möglich, diese Zwischenprodukte der Formel (II) abzutrennen.

Die Reaktionsvariante (b) gemäß der vorliegenden Erfindung wird vorzugsweise in Gegenwart eines Lösungsmittels oder Verdünnungsmittels durchgeführt. Bevorzugte Lösungsmittel oder Verdünnungsmittel in dem Verfahren sind beliebige der inerten Lösungsmittel, wie sie oben für die Reaktionsvariante (a) genannt wurden. In gleicher Weise wird die Reaktionsvariante (b) vorzugsweise in Gegenwart von Säure-Acceptoren durchgeführt, wie sie oben für die Reaktionsvariante (a) genannt wurden.

Die Reaktionsvariante (b) kann innerhalb eines breiten Temperaturbereichs durchgeführt werden, beispielsweise bei einer Temperatur zwischen etwa -20°C und dem Siedepunkt der Reaktionsmischung, vorzugsweise zwischen 0°C und 100°C. Die Reaktionsvariante (b) wird unter normalem (Umgebungs-)Druck durchgeführt, jedoch ist es auch möglich, unter erhöhtem oder vermindertem Druck zu arbeiten.

Die Reaktion zur Herstellung der neuen Zwischenprodukte der allgemeinen Formel (VI), d.h. die Reaktion der Verbindungen der Formel (VIII) mit den Dihalogeno-Verbindungen der Formel (IX) wird ebenfalls vorzugsweise in Gegenwart eines Lösungsmittels oder Verdünnungsmittels durchgeführt. Bevorzugte Lösungsmittel oder Verdünnungsmittel sind beliebige der inerten Lösungsmittel, wie sie oben für die Reaktionsvariante (a) genannt wurden. In gleicher Weise wird die Reaktion vorzugsweise in Gegenwart eines Säure-Acceptors durchgeführt,

wie sie oben für die Reaktionsvariante (a) genannt wurden. Die Temperatur kann innerhalb eines weiten Bereichs gehalten werden, beispielsweise auf einem Temperatur-Wert zwischen -20°C und dem Siedepunkt der Mischung, vorzugsweise zwischen 0°C und 100°C. Die Reaktion wird unter normalem (Umgebungs-)Druck durchgeführt, jedoch ist es auch möglich, unter erhöhtem oder vermindertem Druck zu arbeiten.

Die Carbamoylimidazol-Derivate gemäß der vorliegenden Erfindung zeigen stark ausgeprägte fungizide Effekte auf den Gebieten der Landwirtschaft und des Gartenbaus. Dementsprechend können sie als fungizide Mittel in der Landwirtschaft und im Gartenbau eingesetzt werden.

Fungizide Mittel werden im Pflanzenschutz eingesetzt zur Bekämpfung von
- Plasmodiophoromycetes,
- Oomycetes,
- Chytridiomycetes,
- Zygomycetes,
- Ascomycetes,
- Basidiomycetes und
- Deuteromycetes.

Antibakterielle Mittel werden im Pflanzenschutz eingesetzt zur Bekämpfung von
- Pseudomonadaceae,
- Rhizobiaceae,
- Enterobacteriaceae,
- Corynebacteriaceae und
- Streptomycetaceae.

Einige der Organismen, die fungale und bakterielle Erkrankungen verursachen und unter die oben aufgeführten

Gattungsnamen fallen, seien im Folgenden als Beispiele, nicht jedoch als Einschränkung, genannt:

Species Botrytis
wie beispielsweise Botrytis cinerea;
Species Plasmopara
wie beispielsweise Plasmopara viticola;
Species Uromyces
wie beispielsweise Uromyces appendiculatus;
Species Sphaerotheca
wie beispielsweise Sphaerotheca fuliginea;
Species Venturia
wie beispielsweise Venturia inaequalis;
Species Podosphaera
wie beispielsweise Podosphaera leucotricha;
Species Phytophthora
wie beispielsweise Phytophthora infestans;
Species Erysiphe
wie beispielsweise Erysiphe graminis;
Species Puccinia
wie beispielsweise Puccinia recondita;
Species Fusarium
wie beispielsweise Fusarium culmorum;;
Species Ustilago
wie beispielsweise Ustilago nuda oder Ustilago avenae;
Species Septoria
wie beispielsweise Septoria nodorum;
Species Tilletia
wie beispielsweise Tilletia caries;
Species Xanthomonas
wie beispielsweise Xanthomonas oryzae;
Species Pseudomonas
wie beispielsweise Pseudomonas lacrymans;

Species Piricularia

wie beispielsweise Piricularia oryzae;

Species Pellicularia

wie beispielsweise Pellicularia sasakii;

Species Pyrenophora

wie beispielsweise Pyrenophora teres (Conidia-Form Drechslera, Syn. Helminthosporium);

Species Cochliobolus

wie beispielsweise Cochliobolus sativus Conidia-Form Drechslera, Syn. Helminthosporium);

Species Cercospora

wie beispielsweise Cercospora canescens.

Insbesondere zeigen die Carbamoylimidazol-Derivate der allgemeinen Formel (I) eine herausragende Wirksamkeit gegen

die Brusone-Krankeit bei Reis (Piricularia oryzae);

die Reis-Fleckenkrankheit (Helminthosporium oryzae);

die Bakanae-Krankheit der Reispflanze (Gibberella fujikuroi);

die Stengelfäule (Sclerotinia sclerotiorum);

den Schwarzpilz des Kohles (Alternaria brassicae);

den Pulver-Mehltau (Sphaerotheca fuliginea) und die Graufäule (Botrytis cinerea) verschiedener Nutzpflanzen,

die Brennfleckenkrankheit der Gurke, Melone und dergleichen (Colletotrichum lagenarium) sowie

die Braunfäule der Tomate (Phytophtora infestans).

Als Fungizid für Landwirtschaft und Gartenbau können die Verbindungen gemäß der vorliegenden Erfindung unmittelbar nach Verdünnen mit Wasser oder aber in Form

verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz können diese Präparate entweder unmittelbar oder nach dem Verdünnen mit Wasser auf die gewünschte Konzentration zur Anwendung gebracht.

Die hier genannten landwirtschaftlich unbedenklichen Hilfsstoffe umfassen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser; weiterhin organische Lösungsmittel, beispielsweise Kohlenwasserstoffe /‾z.B. n-Hexan, Petrolether, Erdöl-Fraktionen (z.B. Paraffinwachse, Kerosin, Leichtöle, Mittelöle und Schweröle), Benzol, Toluol und Xylole_7, weiterhin halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkoholether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Schwefel, Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel

vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol und niedere Ether), die Verbrennung steuernde Mittel für Räucherpräparate (z.B. Nitrite, Zink-Pulver und Dicyandiamid), Sauerstoff abgebende Mittel (z.B. Chlorate), wirkungsverlängernde Mittel, Dispersions-Stabilisatoren /¯z.B. Casein, Tragant, Carboxymethylcellulose (CMC) und Polyvinylalkohol (PVA)_7 und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Zu Beispielen für solche Anwendungsformen zählen emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Das landwirtschaftliche und gartenbauliche Fungizid gemäß der vorliegenden Erfindung kann etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Form des Präparats, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Bodenbehandlung (Vermischen mit dem Boden, Streuen, Bedampfen, Gießen etc.), Oberflächenbehandlung (wie Beschichten, Bändern, Bestäuben, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von annähernd 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha.

Gemäß der vorliegenden Erfindung kann ein fungizides Mittel für Landwirtschaft und Gartenbau verfügbar gemacht werden, das eine Verbindung der allgemeinen Formel (I) als Wirkstoff und ein Verdünnungsmittel (ein Lösungsmittel und/oder ein Streckmittel und/oder einen Träger) und/ oder ein oberflächenaktives Mittel und, sofern weiter erforderlich, einen Stabilisator, ein Haftmittel, ein synergistisches Mittel etc. enthält.

Die vorliegende Erfindung stellt ebenfalls ein Verfahren zur Bekämpfung von Pflanzenkrankheiten zur Verfügung, bei dem auf ein Pathogen und/oder den Ort seines Auftretens und/oder den Ort des Auftretens einer Pflanzenerkrankung die Verbindung der Formel (I) entweder einzeln oder im Gemisch mit einem Verdünnungsmittel (einem Lösungsmittel und/ oder einem Streckmittel und/oder einem Träger) und/oder einem oberflächenaktiven Mittel und, sofern weiter erforderlich, einem Stabilisator, einem Haftmittel, einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele:

Beispiel 1

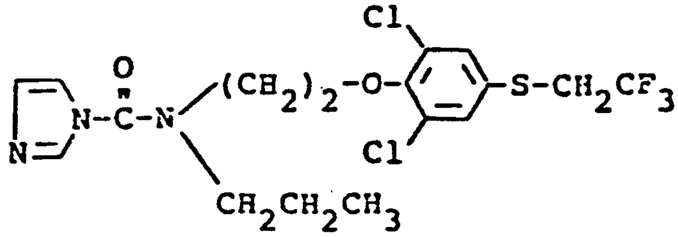

(Verbindung Nr. 1)

Zu 2,16 g rohem N-[2-[2,6-Dichloro-4-(2,2,2-trifluoroethylthio)-phenoxy]ethyl]-N-propylamin wurden 10 ml Toluol und 1,7 g N,N'-Carbonyldiimidazol hinzugefügt. Die Mischung wurde 3 h bei 50°C und danach 6 h unter Rückfluß gerührt. Nach dem Abkühlen wurden 60 ml Toluol zugegeben. Die Mischung wurde mit Wasser gewaschen und über wasserfreiem Natriumsulfat getrocknet. Das Abdampfen des Toluols lieferte 2,2 g 1-[N-2-[2,6-Dichloro-4-(2,2,2-trifluoroethylthio)-phenoxy]ethyl-N-propyl-carbamoyl]imidazol mit dem Brechungsindex $n_D^{20}$ = 1,5491.

Die folgenden Verbindungen der allgemeinen Formel (I)

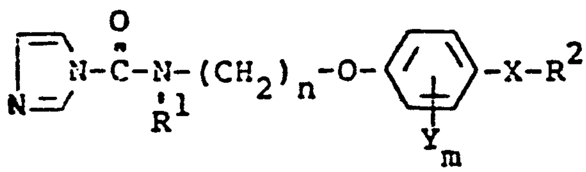

(I)

wurden nach der in Beispiel 1 beschriebenen Arbeitsweise erhalten.

| Verbindung Nr. | $R^1$ | n | $Y_m$ | $X-R^2$ | Physikal. Konstante |
|---|---|---|---|---|---|
| 2 | $-CH_3$ | 2 | - | $-SCH_2CF_3$ | $n_D^{20}$ 1.5462 |
| 3 | $-C_2H_5$ | 2 | - | $-SCH_2CF_3$ | $n_D^{20}$ 1.5395 |
| 4 | $-C_3H_7$-iso | 2 | - | $-SCH_2CF_3$ | $n_D^{20}$ 1.5314 |
| 5 | $-C_3H_7$-n | 2 | - | $-SCH_2CF_3$ | $n_D^{20}$ 1.5332 |
| 6 | $-C_4H_9$-sec | 2 | - | $-SCH_2CF_3$ | $n_D^{20}$ 1.5295 |
| 7 | $-C_4H_9$-n | 2 | - | $-SCH_2CF_3$ | $n_D^{20}$ 1.5256 |
| 8 | $-C_2H_4OC_2H_5$ | 2 | - | $-SCH_2CF_3$ | $n_D^{20}$ 1.5274 |
| 9 | 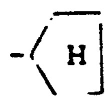 | 2 | - | $-SCH_2CF_3$ | $n_D^{20}$ 1.5460 |
| 10 | $-C_3H_7$-n | 2 | $2-CH_3$ | $-SCH_2CF_3$ | $n_D^{20}$ 1.5345 |
| 11 | $-C_4H_9$-sec | 2 | $2-CH_3$ | $-SCH_2CF_3$ | $n_D^{20}$ 1.5313 |
| 12 | $-C_4H_9$-sec | 2 | $3,5-(CH_3)_2$ | $-SCH_2CF_3$ | $n_D^{20}$ 1.5330 |
| 13 | $-C_3H_7$-n | 2 | 2-Cl | $-SCH_2CF_3$ | $n_D^{20}$ 1.5462 |
| 14 | $-C_4H_9$-sec | 2 | 2-Cl | $-SCH_2CF_3$ | $n_D^{20}$ 1.5420 |
| 15 | $-C_4H_9$-sec | 2 | $2,6-Cl_2$ | $-SCH_2CF_3$ | $n_D^{20}$ 1.5460 |
| 16 | 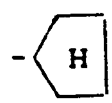 | 2 | $2,6-Cl_2$ | $-SCH_2CF_3$ | $n_D^{20}$ 1.5592 |
| 17 | $-C_3H_7$-n | 3 | - | $-SCH_2CF_3$ | $n_D^{20}$ 1.5310 |
| 18 | $-C_4H_9$-sec | 3 | - | $-SCH_2CF_3$ | $n_D^{20}$ 1.5260 |

| Verbindung Nr. | $R^1$ | n | $Y_m$ | $X-R^2$ | Physikal. Konstante |
|---|---|---|---|---|---|
| 19 | - 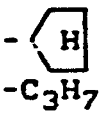 | 3 | - | $-SCH_2CF_3$ | $n_D^{20}$ 1.5415 |
| 20 | $-C_3H_7-n$ | 3 | $2.6-Cl_2$ | $-SCH_2CF_3$ | $n_D^{20}$ 1.5498 |
| 21 | $-C_4H_9$-sec | 3 | $2.6-Cl_2$ | $-SCH_2CF_3$ | $n_D^{20}$ 1.5430 |
| 22 | - 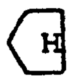 | 3 | $2.6-Cl_2$ | $-SCH_2CF_3$ | $n_D^{20}$ 1.5570 |
| 23 | $-C_3H_7-n$ | 4 | - | $-SCH_2CF_3$ | $n_D^{20}$ 1.5260 |
| 24 | $-C_4H_9$-sec | 4 | - | $-SCH_2CF_3$ | $n_D^{20}$ 1.5252 |
| 25 | - 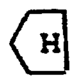 | 4 | - | $-SCH_2CF_3$ | $n_D^{20}$ 1.5390 |
| 26 | $-C_3H_7-n$ | 4 | - | $-SCH_2CF_3$ | $n_D^{20}$ 1.5280 |
| 27 | $-C_4H_9$-sec | 4 | $2-CH_3$ | $-SCH_2CF_3$ | $n_D^{20}$ 1.5250 |
| 28 | $-C_3H_7-n$ | 4 | $3,5-(CH_3)_2$ | $-SCH_2CF_3$ | $n_D^{20}$ 1.5300 |
| 29 | $-C_4H_9$-sec | 4 | $3,5-(CH_3)_2$ | $-SCH_2CF_3$ | $n_D^{20}$ 1.5266 |
| 30 | $-C_3H_7-n$ | 4 | 2-Cl | $-SCH_2CF_3$ | $n_D^{20}$ 1.5380 |
| 31 | $-C_4H_9$-sec | 4 | 2-Cl | $-SCH_2CF_3$ | $n_D^{20}$ 1.5358 |
| 32 | $-C_3H_7-n$ | 5 | - | $-SCH_2CF_3$ | $n_D^{20}$ 1.5260 |
| 33 | $-C_4H_9$-sec | 5 | - | $-SCH_2CF_3$ | $n_D^{20}$ 1.5245 |
| 34 | $-C_4H_9$-sec | 5 | $2,6-Cl_2$ | $-SCH_2CF_3$ | $n_D^{20}$ 1.5395 |
| 35 | $-C_3H_7-n$ | 6 | - | $-SCH_2CF_3$ | $n_D^{20}$ 1.5245 |
| 36 | $-C_3H_7-n$ | 2 | - | $-SCF_3$ | Schmp. 81-82°C |
| 37 | $-C_4H_9$-sec | 2 | - | $-SCF_3$ | $n_D^{20}$ 1.5247 |

| Verbindung Nr. | $R^1$ | n | $Y_m$ | $X-R^2$ | Physikal. Konstante |
|---|---|---|---|---|---|
| 38 | -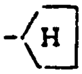 | 2 | - | $-SCF_3$ | Schmp. 90-91°C |
| 39 | 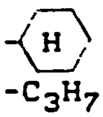 | 2 | - | $-SCF_3$ | $n_D^{20}$ 1.5375 |
| 40 | $-C_3H_7$-n | 2 | 2-Cl | $-SCF_3$ | Schmp. 98-99°C |
| 41 | $-C_4H_9$-sec | 2 | 2-Cl | $-SCF_3$ | $n_D^{20}$ 1.5240 |
| 42 | $-C_3H_7$-n | 2- | $2,6-Cl_2$ | $-SCF_3$ | $n_D^{20}$ 1.5410 |
| 43 | $-C_4H_9$-sec | 2 | $2,6-Cl_2$ | $-SCF_3$ | $n_D^{20}$ 1.5188 |
| 44 | -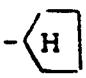 | 2 | $2,6-Cl_2$ | $-SCF_3$ | $n_D^{20}$ 1.5483 |
| 45 | $-C_3H_7$-n | 3 | - | $-SCF_3$ | $n_D^{20}$ 1.5220 |
| 46 | $-C_4H_9$-sec | 3 | - | $-SCF_3$ | $n_D^{20}$ 1.5142 |
| 47 | -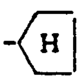 | 3 | - | $-SCF_3$ | Schmp. 83-84°C |
| 48 | $-C_3H_7$-n | 3 | 2-Cl | $-SCF_3$ | Schmp. 101-102°C |
| 49 | $-C_4H_9$-sec | 3 | 2-Cl | $-SCF_3$ | $n_D^{20}$ 1.5284 |
| 50 | $-C_3H_7$-n | 3 | $2,6-Cl_2$ | $-SCF_3$ | $n_D^{20}$ 1.5390 |
| 51 | $-C_4H_9$-sec | 3 | $2,6-Cl_2$ | $-SCF_3$ | $n_D^{20}$ 1.5341 |
| 52 | -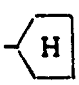 | 3 | $2,-Cl_2$ | $-SCF_3$ | Schmp. 92-93°C |
| 53 | $-C_3H_7$-n | 4 | - | $-SCF_3$ | $n_D^{20}$ 1.5225 |
| 54 | $-C_4H_9$-sec | 4 | - | $-SCF_3$ | $n_D^{20}$ 1.5170 |

| Verbindung Nr. | $R^1$ | n | $Y_m$ | $X-R^2$ | Physikal. Konstante |
|---|---|---|---|---|---|
| 55 | 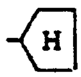 | 4 | - | $-SCF_3$ | $n_D^{20}$ 1.5330 |
| 56 | $-C_3H_7$-n | 4 | 2-Cl | $-SCF_3$ | $n_D^{20}$ 1.5310 |
| 57 | $-C_4H_9$-sec | 4 | 2-Cl | $-SCF_3$ | $n_D^{20}$ 1.5298 |
| 58 | $-C_3H_7$-n | 5 | - | $-SCF_3$ | $n_D^{20}$ 1.5209 |
| 59 | $-C_4H_9$-sec | 5 | - | $-SCF_3$ | $n_D^{20}$ 1.5186 |
| 60 | 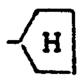 | 5 | - | $-SCF_3$ | $n_D^{20}$ 1.5303 |
| 61 | $-C_3H_7$-n | 5 | 2-Cl | $-SCF_3$ | $n_D^{20}$ 1.5291 |
| 62 | $-C_4H_9$-sec | 5 | 2-Cl | $-SCF_3$ | $n_D^{20}$ 1.5273 |
| 63 | $-C_4H_9$-sec | 5 | 2,6-$Cl_2$ | $-SCF_3$ | $n_D^{20}$ 1.5323 |
| 64 | $-C_3H_7$-n | 2 | - | $-OCF_3$ | $n_D^{20}$ 1.5000 |
| 65 | $-C_4H_9$-sec | 2 | - | $-OCF_3$ | $n_D^{20}$ 1.4963 |
| 66 | $-C_3H_7$-n | 2 | 2-Cl | $-OCF_3$ | Schmp. 77-78°C |
| 67 | $-C_4H_9$-sec | 2 | 2-Cl | $-OCF_3$ | $n_D^{20}$ 1.5044 |
| 68 | $-C_3H_7$-n | 2 | 2,6-$Cl_2$ | $-OCF_3$ | $n_D^{20}$ 1.5163 |
| 69 | $-C_4H_9$-sec | 2 | 2,6-$Cl_2$ | $-OCF_3$ | $n_D^{20}$ 1.5122 |
| 70 | 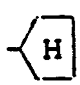 | 2 | 2,6-$Cl_2$ | $-OCF_3$ | $n_D^{20}$ 1.5275 |
| 71 | $-C_3H_7$-n | 3 | - | $-OCF_3$ | $n_D^{20}$ 1.4978 |
| 72 | $-C_4H_9$-sec | 3 | - | $-OCF_3$ | $n_D^{20}$ 1.4947 |
| 73 | $-C_3H_7$-n | 3 | 2-Cl | $-OCF_3$ | $n_D^{20}$ 1.5097 |
| 74 | $-C_4H_9$-sec | 3 | 2-Cl | $-OCF_3$ | $n_D^{20}$ 1.5057 |

| Verbindung Nr. | $R^1$ | n | $Y_m$ | $X-R^2$ | Physikal. Konstante |
|---|---|---|---|---|---|
| 75 | $-C_3H_7-n$ | 3 | 2,6-$Cl_2$ | $-OCF_3$ | $n_D^{20}$ 1.5146 |
| 76 | $-C_4H_9-sec$ | 3 | 2,6-$Cl_2$ | $-OCF_3$ | $n_D^{20}$ 1.5112 |
| 77 | 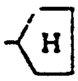 | 3 | 2,6-$Cl_2$ | $-OCF_3$ | Schmp. 80-82°C |
| 78 | $-C_3H_7-n$ | 5 | - | $-OCF_3$ | $n_D^{20}$ 1.4955 |
| 79 | $-C_4H_9-sec$ | 5 | - | $-OCF_3$ | $n_D^{20}$ 1.4942 |
| 80 | $-C_3H_7-n$ | 5 | 2-Cl | $-OCF_3$ | $n_D^{20}$ 1.5050 |
| 81 | $-C_4H_9-sec$ | 5 | 2-Cl | $-OCF_3$ | $n_D^{20}$ 1.5036 |
| 82 | $-C_3H_7-n$ | 5 | 2,6-$Cl_2$ | $-OCF_3$ | $n_D^{20}$ 1.5096 |
| 83 | $-C_4H_9-sec$ | 5 | 2,6-$Cl_2$ | $-OCF_3$ | $n_D^{20}$ 1.5075 |
| 84 | 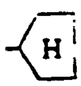 | 5 | 2,6-$Cl_2$ | $-OCF_3$ | $n_D^{20}$ 1.5160 |

Anmerkung:

Das Symbol " - " in der Spalte $Y_m$ bedeutet, daß kein Substituent Y vorhanden ist und m die Bedeutung 0 (Null) hat.

Herstellung der Ausgangsstoffe:
(Herstellung der Verbindungen der allgemeinen Formeln II, VI und VIII).

Beispiel II-1

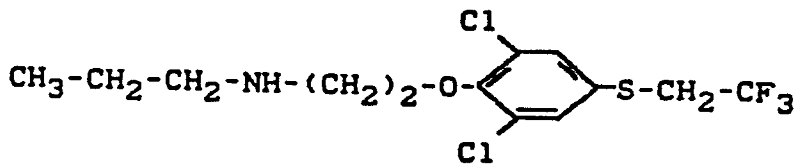

5 g n-Propylamin wurden zu 2,38 g 2-/¯2,6-Dichloro-4-(2,2,2-trifluoroethylthio)-phenoxy_7ethylbromid hinzugefügt, und die Mischung wurde 8 h bei Raumtemperatur und dann 3 h unter Rückfluß gerührt. Der Überschuß an n-Propylamin wurde abgedampft. Dem Rückstand wurden 20 ml Wasser, 20 ml einer 5-proz. wäßrigen Kaliumhydroxid-Lösung und 100 ml Toluol zugesetzt. Die Mischung wurde gerührt, und die organische Schicht wurde abgetrennt, mit Wasser gewaschen und über wasserfreiem Natriumsulfat getrocknet. Die niedrigsiedenden Komponenten wurden unter vermindertem Druck abgedampft. Erhalten wurden 2,16 g rohes N-/=2-/¯2,6-Dichloro-4-(2,2,2-trifluoroethylthio)-phenoxy_7ethyl_7-N-propylamin mit einem Brechungsindex $n_D^{20}$ = 1,5210, das ohne weitere Reinigung für die Herstellung der oben bezeichneten Verbindung Nr. 1 verwendet wurde.

Beispiel VI-1

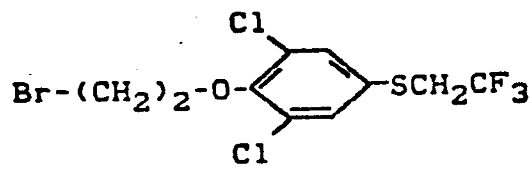

Zu einer durch Auflösen von 0,7 g metallischem Natrium in 50 ml Ethanol erhaltenen ethanolischen Natriumethylat-Lösung wurden 8,9 g 2,6-Dichloro-4-(2,2,2-trifluoroethylthio)phenol und 20 g Ethylenbromid hinzugefügt. Die Mischung wurde 2 h bei 40°C und dann 4 h unter Rückfluß gerührt. Ethanol und der Überschuß an Ethylenbromid wurden abgedampft. Der Rückstand wurde in 150 ml Toluol aufgenommen, mit einer 5-proz. wäßrigen Kaliumhydroxid-Lösung und mit Wasser in dieser Reihenfolge gewaschen und über wasserfreiem Natriumsulfat getrocknet. Das Toluol wurde abgedampft, und der Rückstand wurde unter vermindertem Druck destilliert, wonach 7,2 g 2-[2,6-Dichloro-4-(2,2,2-trifluoroethylthio)-phenoxy]ethylbromid mit dem Sdp. 124-126°C/1,07 mbar erhalten wurden.

Die folgenden Verbindungen der Formel (VIa) (das heißt, Verbindungen der allgemeinen Formel (VI), in der "Hal" Br ist)

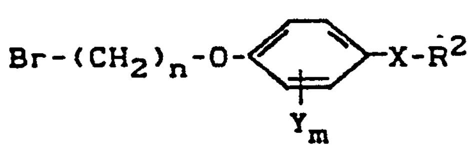 (VIa)

wurden nach der in Beispiel VI-1 beschriebenen Verfahrensweise erhalten:

Nit 184

| Verbindung | n | $Y_m$ | $X-R^2$ | Sdp.°C/mbar |
|---|---|---|---|---|
| VI-2 | 2 | - | $-SCH_2CF_3$ | 136-139/13,3 |
| VI-3 | 3 | - | $-SCH_2CF_3$ | 140-144/13,3 |
| VI-4 | 4 | - | $-SCH_2CF_3$ | 143-145/ 6,67 |
| VI-5 | 5 | - | $-SCH_2CF_3$ | 135-140/ 2,0 |
| VI-6 | 6 | - | $-SCH_2CF_3$ | 145-150/ 2,0 |
| VI-7 | 2 | $2-CH_3$ | $-SCH_2CF_3$ | 114-116/ 1,07 |
| VI-8 | 4 | $2-CH_3$ | $-SCH_2CF_3$ | 126-129/ 1,07 |
| VI-9 | 2 | $3,5-(CH_3)_2$ | $-SCH_2CF_3$ | 123-125/ 2,7 |
| VI-10 | 4 | $3,5-(CH_3)_2$ | $-SCH_2CF_3$ | 132-134/ 1,33 |
| VI-11 | 2 | 2-Cl | $-SCH_2CF_3$ | 118-120/ 1,07 |
| VI-12 | 4 | 2-Cl | $-SCH_2CF_3$ | 135-137/ 1,33 |
| VI-13 | 2 | $2,6-Cl_2$ | $-SCH_2CF_3$ | 124-126/ 1,07 |
| VI-14 | 3 | $2,6-Cl_2$ | $-SCH_2CF_3$ | 151-153/ 2,0 |
| VI-15 | 4 | $2,6-Cl_2$ | $-SCH_2CF_3$ | 137-139/ 1,07 |
| VI-16 | 5 | $2,6-Cl_2$ | $-SCH_2CF_3$ | 143-145/ 0,8 |
| VI-17 | 2 | - | $-OCF_3$ | 90-91/ 0,53 |
| VI-18 | 3 | - | $-OCF_3$ | 105-106/ 5,1 |
| VI-19 | 5 | - | $-OCF_3$ | 133-135/ 4,9 |
| VI-20 | 2 | 2-Cl | $-OCF_3$ | 105-107/ 4,9 |
| VI-21 | 3 | 2-Cl | $-OCF_3$ | 118-119/ 4,9 |
| VI-22 | 5 | 2-Cl | $-OCF_3$ | 144-146/ 4,9 |
| VI-23 | 2 | $2,6-Cl_2$ | $-OCF_3$ | 111-113/ 4,9 |
| VI-24 | 3 | $2,6-Cl_2$ | $-OCF_3$ | 125-127/ 4,9 |
| VI-25 | 4 | $2,6-Cl_2$ | $-OCF_3$ | 136-138/ 4,9 |
| VI-26 | 5 | $2,6-Cl_2$ | $-OCF_3$ | 145-148/ 4,9 |
| VI-27 | 2 | - | $-SCF_3$ | 85-90/ 1,00 |
| VI-28 | 3 | - | $-SCF_3$ | 105-108/ 1,10 |
| VI-29 | 4 | - | $-SCF_3$ | 105-108/ 1,00 |
| VI-30 | 5 | - | $-SCF_3$ | 115-120/ 1,00 |
| VI-31 | 2 | 2-Cl | $-SCF_3$ | 105-106/ 1,00 |

| Verbindung | n | $Y_m$ | $X-R^2$ | Sdp.°C/mbar |
|---|---|---|---|---|
| VI-32 | 3 | 2-Cl | $-SCF_3$ | 116-117/ 0,91 |
| VI-33 | 4 | 2-Cl | $-SCF_3$ | 134-136/ 0,8 |
| VI-34 | 5 | 2-Cl | $-SCF_3$ | 140-142/ 1,00 |
| VI-35 | 2 | 2,6-$Cl_2$ | $-SCF_3$ | 114-116/ 1,00 |
| VI-36 | 3 | 2,6-$Cl_2$ | $-SCF_3$ | 113-120/ 0,8 |
| VI-37 | 4 | 2,6-$Cl_2$ | $-SCF_3$ | 130-132/ 1,00 |
| VI-38 | 5 | 2,6-$Cl_2$ | $-SCF_3$ | 142-145/ 0,85 |

Anmerkung:
Das Symbol " - " in der Spalte $Y_m$ bedeutet, daß kein Substituent Y vorhanden ist und m die Bedeutung 0 (Null) hat.

Beispiel VIII-1

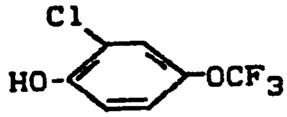

Gasförmiges Chlor wurde langsam in eine gerührte Lösung von 35,6 g 4-Trifluoromethoxyphenol in 100 ml Kohlenstofftetrachlorid eingeleitet. Die Reaktionstemperatur wurde mittels Außenkühlung auf 20°C bis 30°C gehalten. Nach dem Sättigen mit Chlor wurde die Reaktionsmischung

1 h bei 20°C bis 30°C gerührt, gekühlt, mit Wasser gewaschen, über wasserfreiem Natriumsulfat getrocknet und eingedampft, wonach ein blaßgelber Rückstand erhalten wurde. Der Rückstand wurde unter vermindertem Druck destilliert, wonach 29,8 g 2-Chloro-4-trifluoromethoxyphenol erhalten wurden, das bei 67-70°C/24 mbar siedete.

Beispiel VIII-2

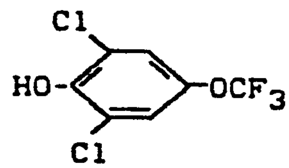

In eine siedende Lösung von 21,3 g 2-Chloro-4-trifluoromethoxyphenol, 2 g wasserfreiem Eisen(III)-chlorid und 100 ml Kohlenstofftetrachlorid wurden im Laufe von 1 h mehr als 8 g gasförmiges Chlor eingeleitet. Die Reaktionsmischung wurde noch 1 h gekocht, abgekühlt, mit Wasser gewaschen, über wasserfreiem Natriumsulfat getrocknet und eingedampft, wonach ein blaßgelber Rückstand erhalten wurde. Der Rückstand wurde unter vermindertem Druck (24 mbar) destilliert, und 9 g einer bei 96-99°C siedenden Fraktion wurden gesammelt. Beim Abkühlen erstarrte die Fraktion. Sie schmolz bei 42-44°C.

Beispiel VIII-3

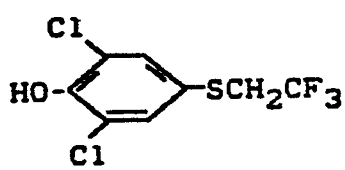

64 g Kaliumcarbonat wurden zu einer Lösung von 41,9 g 3,5-Dichloro-4-hydroxybenzolthiophenol in 200 ml N,N-Dimethylformamid unter einer Stickstoff-Atmopshäre hinzugefügt. Zu der gerührten Mischung wurden bei 30°C bis 40°C 55 g 2,2,2-Trifluoroethylbenzolsulfonat zugetropft. Das Ganze wurde 6 h bei 60°C bis 70°C gerührt, abgekühlt, mit 1,5 l Wasser vermischt und durch tropfenweise Zugabe von Salzsäure auf pH 2 angesäuert. Das anfallende Öl wurde mit Toluol extrahiert, und der Toluol-Extrakt wurde mit Wasser gewaschen. Der gewaschene Toluol-Extrakt wurde mit 160 ml einer 25-proz. wäßrigen Kaliumhydroxid-Lösung gewaschen, und die wäßrige Schicht wurde abgetrennt. Dann wurde die abgetrennte Wasser-Schicht durch Zusatz von Salzsäure auf pH 2 angesäuert. Ein dabei anfallendes Öl wurde mit Toluol extrahiert. Der Toluol-Extrakt wurde mit Wasser gewaschen, über wasserfreiem Natriumsulfat getrocknet und eingedampft, wonach ein Rückstand erhalten wurde. Der Rückstand wurde unter vermindertem Druck destilliert, wonach 42,3 g 2,6-Dichloro-4-(2,2,2-trifluoroethylthio)phenol erhalten wurden, das bei 85-88°C/1,33 mbar siedete.

Beispiel VIII-4

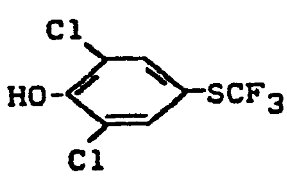

In der gleichen Weise, wie in Beispiel VIII-3 angegeben ist, wurde unter Einsatz von Trifluoroiodomethan an Stelle von 2,2,2-Trifluoroethylbenzolsulfonat 2,6-Dichloro-4-trifluoromethylphenol mit dem Sdp. 110-112°C/24 mbar und dem Schmp. 38-40°C hergestellt.

Verwendungsbeispiele:

Die bekannten Vergleichs-Verbindungen sind wie folgt identifiziert:

Vergleichs-Verbindung A-1:

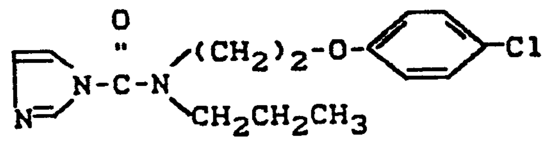

Vergleichs-Verbindung A-2

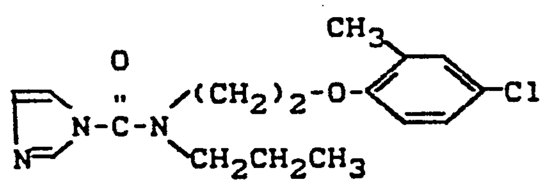

Beide der im Vorstehenden bezeichneten Verbindungen sind in der JP-OS 31047/1975 bzw. der GB-PS 1 469 779 beschrieben.

Beispiel A

Test auf Wirksamkeit gegen die Brusone-Krankheit von Reis (Piricularia oryzae) bei Sprüh-Behandlung von Stengeln und Blättern:

Herstellung einer Test-Verbindung:

| | | |
|---|---|---|
| Wirkstoff: | 50 Gew.-Teile; | |
| Träger: | 45 Gew.-Teile einer Mischung (1 : 5) aus Diatomeenerde und Kaolin; | |
| Emulgator: | 5 Gew.-Teile Polyoxyethylenalkylphenylether. | |

Die aktive Verbindung, der Träger und der Emulgator wurden in den angegebenen Mengen pulverisiert und miteinander vermischt, wodurch ein benetzbares Pulver gebildet wurde. Das Test-Präparat wurde dadurch hergestellt, daß eine vorher festgelegte Menge des benetzbaren Pulvers mit Wasser verdünnt wurde.

Test-Verfahren

Reispflanzen (Varietät: Asahi) wurden in unglasierten Töpfen mit einem Durchmesser von 12 cm kultiviert, und im 3- bis 4-Blatt-Stadium wurde die wie oben auf eine vorher festgelegte Konzentration gebrachte Test-Verbindung in einer Menge von 50 ml auf jeweils drei Töpfe aufgesprüht. Am nächsten Tag wurde eine Suspension künstlich gezüchteter Sporen von Piricularia oryzae durch (zweimaliges) Sprühen inokuliert. Zur Infizierung der Pflanzen mit dem Pilz wurden die Töpfe in einem feuchten Raum bei einer Temperatur von 25°C und einer relativen Luftfeuchtigkeit von 100 % gehalten. Sieben Tage nach der Inokulation wurde der Grad der Erkrankung jedes Topfes untersucht und aufgrund des folgenden Bewertungsmaßstabs beurteilt. Der Bekämpfungs-Index (%) wurde berechnet. Die Phytotoxizität wurde ebenfalls geprüft.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

Im vorliegenden Test bildeten drei Töpfe eine Fläche. Die Ergebnisse sind in der folgenden Tabelle aufgeführt.

Tabelle A

Test auf Wirksamkeit gegen die Brusone-Krankheit von Reis (Piricularia oryzae)

| Verbindung Nr. | Wirkstoff-Konzentration ppm | Bekämpfungs-Index (%) |
|---|---|---|
| A-1 (Vergleichs-Verbindung) | 250 | 40 |
| 1 | 250 | 100 |
| 16 | 250 | 100 |
| 18 | 250 | 100 |
| 21 | 250 | 100 |
| 23 | 250 | 100 |
| 31 | 250 | 100 |
| 40 | 250 | 100 |
| 42 | 250 | 100 |
| 46 | 250 | 100 |
| 65 | 250 | 100 |
| 68 | 250 | 100 |

Beispiel B

Test auf Wirksamkeit gegen die Braunfäule der Tomate (Phytophthora infestans):

Jede der Test-Verbindungen in Form einer Emulsion, die gemäß dem nachstehenden Beispiel (b) hergestellt worden war, wurde mit einer Sprühpistole auf in unglasierten 9 cm-Töpfen gezogene Tomaten (Varietät: "Kurihara") aufgesprüht. Einen Tag nach dem Sprühen wurden die Pflanzen mit einer Sporen-Suspension des oben bezeichneten Pathogens inokuliert, und die Töpfe wurden über Nacht in einem Raum mit einer konstanten Temperatur von 22°C und einer Luftfeuchtigkeit von wenigstens 90 % stehen gelassen. Fünf Tage später wurde der Grad der Erkrankung aufgrund des nachfolgenden Bewertungsmaßstabs durch das prozentuale Verhältnis der Läsionen zeigenden Flächen bestimmt, und der Bekämpfungsindex wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

Die Ergebnisse sind in der folgenden Tabelle aufgeführt.

Tabelle B

Test auf Wirksamkeit gegen die Braunfäule der Tomate (Phytophthora infestans):

| Verbindung Nr. | Wirkstoff-Konzentration (ppm) | Bekämpfungs-Index (%) |
|---|---|---|
| A-1 | 1000 | 35 |
| | 500 | 0 |
| (Vergleichs-Verbindung) | | |
| A-2 | 1000 | 40 |
| | 500 | 0 |
| (Vergleichs-Verbindung) | | |
| 6 | 500 | 100 |
| 23 | 500 | 100 |
| 25 | 500 | 100 |
| 32 | 500 | 100 |
| 36 | 500 | 100 |
| 45 | 500 | 100 |
| 66 | 500 | 100 |

Beispiel C

Test auf Wirksamkeit gegen die Brennfleckenkrankheit der Gurke (Colletotrichum lagenarium):

Jede der Test-Verbindungen in Form einer Emulsion, die gemäß dem nachstehenden Beispiel (b) hergestellt worden war, wurde mit einer Sprühpistole auf in unglasierten 9 cm-Töpfen gezogene Gurken (Varietät: "Yotsuba") im 2-Blatt-Stadium aufgesprüht. Einen Tag nach dem Sprühen wurden die Pflanzen mit einer Sporen-Suspension des oben bezeichneten Pathogens (Colletotrichum lagenarium) durch Sprühen inokuliert, und die Töpfe wurden über Nacht in einem Raum mit einer konstanten Temperatur von 22°C und einer Luftfeuchtigkeit von wenigstens 90 % stehen gelassen. Sechs Tage später wurde der Grad der Erkrankung aufgrund des nachfolgenden Bewertungsmaßstabs durch das prozentuale Verhältnis der Läsionen zeigenden Flächen wie in Beispiel B bestimmt, und der Bekämpfungsindex wurde ebenfalls berechnet.

Die Ergebnisse sind in der folgenden Tabelle aufgeführt.

Tabelle C

Test auf Wirksamkeit gegen die Brennfleckenkrankheit der Gurke (Colletotrichum lagenarium):

| Verbindung Nr. | Wirkstoff-Konzentration ppm | Bekämpfungs-Index (%) |
|---|---|---|
| A-2 (Vergleichs-Verbindung) | 250 | 35 |
| 1 | 250 | 100 |
| 23 | 250 | 100 |
| 31 | 250 | 100 |
| 32 | 250 | 100 |
| 36 | 250 | 100 |
| 40 | 250 | 100 |
| 65 | 250 | 100 |
| 68 | 250 | 100 |
| 69 | 250 | 100 |
| 73 | 250 | 100 |
| 75 | 250 | 100 |
| 78 | 250 | 100 |
| 79 | 250 | 100 |
| 81 | 250 | 100 |

Beispiel D

Test auf Wirksamkeit gegen den Pulver-Mehltau der Gurke (Sphaerotheca fuliginea):

Jede der Test-Verbindungen in Form einer Emulsion, die gemäß dem nachstehenden Beispiel (b) hergestellt worden war, wurde mit einer Sprühpistole auf in unglasierten 9 cm-Töpfen gezogene Gurken (Varietät: "Tokiwa", kriechender Typ) im 2-Blatt-Stadium aufgesprüht. Einen Tag nach dem Sprühen wurden die Pflanzen mit einer Sporen-Suspension des oben bezeichneten Pathogens (Sphaerotheca fuliginea) durch Sprühen inokuliert, und die Töpfe wurden über Nacht in einem Raum mit einer konstanten Temperatur von 23°C stehen gelassen. Zehn Tage später wurde der Grad der Erkrankung aufgrund des nachfolgenden Bewertungsmaßstabs durch das prozentuale Verhältnis der Läsionen zeigenden Flächen wie in Beispiel B bestimmt, und der Bekämpfungsindex wurde ebenfalls berechnet.

Die Ergebnisse sind in der folgenden Tabelle aufgeführt.

Tabelle D

Test auf Wirksamkeit gegen den Pulver-Mehltau der Gurke (Sphaerotheca fuliginea):

| Verbindung Nr. | Wirkstoff-Konzentration ppm | Bekämpfungs-Index (%) |
|---|---|---|
| A-1 (Vergleichs-Verbindung) | 100 | 40 |
| A-2 | 50 | 0 |
| | 100 | 0 |
| (Vergleichs-Verbindung) | | |
| 6 | 50 | 100 |
| 7 | 50 | 100 |
| 37 | 50 | 100 |
| 41 | 50 | 100 |
| 42 | 50 | 100 |
| 43 | 50 | 100 |
| 44 | 50 | 100 |
| 46 | 50 | 100 |
| 50 | 50 | 100 |
| 51 | 50 | 100 |
| 52 | 50 | 100 |
| 53 | 50 | 100 |
| 54 | 50 | 100 |
| 58 | 50 | 100 |
| 59 | 50 | 100 |
| 62 | 50 | 100 |
| 63 | 50 | 100 |
| 68 | 50 | 100 |

Nit 184

Beispiel E

Test auf Wirksamkeit gegen die Reis-Fleckenkrankheit (Helminthosporium oryzae) bei Sprüh-Behandlung von Stengeln und Blättern:

Reispflanzen (Varietät: Kusabue) wurden in unglasierten Töpfen mit einem Durchmesser von 12 cm kultiviert, und im 3- bis 4-Blatt-Stadium wurde eine Verdünnung jeder der Test-Verbindungen, die gemäß dem nachstehenden Beispiel (a) hergestellt worden war, in einer Menge von 50 ml auf jeweils drei Töpfe aufgesprüht. Am nächsten Tag wurde eine Suspension künstlich kultivierter Sporen von Helminthosporium oryzae zweimal auf die Töpfe gesprüht. Zur Induzierung der Infektion wurden die Töpfe in einer feuchten Kammer bei einer relativen Luftfeuchtigkeit von 100 % und einer Temperatur von 25°C gehalten. Sieben Tage nach der Inokulation wurden der Grad der Erkrankung jedes Topfes untersucht und aufgrund des folgenden Bewertungsmaßstabs mit den Noten 0 bis 5 bewertet, und der Bekämpfungs-Index (%) wurde berechnet.

| Grad der Erkrankung | Ausmaß der Erkrankung |
|---|---|
| 0 | keine Erkrankung |
| 1 | nur geringfügig |
| 2 | leicht |
| 3 | mittel |
| 4 | schwer |
| 5 | sehr schwer |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

Im vorliegenden Test bildeten drei Töpfe eine Fläche. Die Ergebnisse sind in der folgenden Tabelle aufgeführt.

Tabelle E

Test auf Wirksamkeit gegen die Reis-Fleckenkrankheit (Helminthosporium oryzae) bei Sprüh-Behandlung von Stengeln und Blättern

| Verbindung Nr. | Wirkstoff-Konzentration ppm | Bekämpfungs-Index (%) |
|---|---|---|
| A-1 | 250 | 50 |
| | 100 | 10 |
| (Vergleichs-Verbindung) | | |
| A-2 | 250 | 50 |
| | 100 | 10 |
| (Vergleichs-Verbindung) | | |
| 1 | 100 | 100 |
| 6 | 100 | 100 |
| 36 | 100 | 100 |
| 42 | 100 | 100 |
| 48 | 100 | 100 |
| 64 | 100 | 100 |
| 68 | 100 | 100 |

Beispiel F

Test auf Wirksamkeit gegen die Reis-Fleckenkrankheit (Helminthosporium oryzae) bei Samen-Behandlung:

Samen (Varietät: Koshihikari), die hochgradig spontan mit der Reis-Fleckenkrankheit (Helminthosporium oryzae) infiziert worden waren, wurden jeweils mit einer Verdünnung der Test-Verbindungen mit vorher festgelegter Konzentration, die gemäß dem nachstehenden Beispiel (a) hergestellt worden war, mit Hilfe der in der folgenden Tabelle angegebenen Methoden behandelt. Am nächsten Tag wurden die behandelten Samenkörner in Setzkästen eingesät und nach der üblichen Methode angezogen. Fünfzehn Tage nach der Einsaat wurden sämtliche Pflänzchen herausgezogen und in erkrankte Sämlinge und gesunde Sämlinge aufgeteilt. Der Bekämpfungs-Index wurde nach der nachstehenden Gleichung aus dem Prozentsatz der gesunden Sämlinge bezogen auf die Gesamtzahl der Sämlinge bezogen auf die Gesamtzahl der eingesäten Samenkörner berechnet. Die Ergebnisse sind in der folgenden Tabelle F aufgeführt.

$$\text{Bekämpfungs-Index (\%)} = 100 - \left[ \frac{100 - \text{Prozentsatz der gesunden Sämlinge auf der behandelten Fläche}}{100 - \text{Prozentsatz der gesunden Sämlinge auf der unbehandelten Fläche}} \times 100 \right]$$

In der Tabelle sind die Behandlungsmethoden wie folgt bezeichnet:

M-1: Die Samen wurden 1/6 h eingetaucht.

M-2: Die Samen wurden 20 h eingetaucht.

M-3: Die Samen wurden mit der pulverförmigen Verbindung bedeckt.

Nit 184

Tabelle F

Test auf Wirksamkeit gegen die Reis-Fleckenkrankheit (Helminthosporium oryzae) bei Samen-Behandlung:

| Verbindung Nr. | Wirkstoff-Konzentration | Test-Methode | Prozentuale Keimung (%) | Prozentsatz der erkrankten Sämlinge bezogen auf die gekeimten Samen (%) | Prozentsatz der gesunden Sämlinge bezogen auf die eingesäten Samen (%) | Bekämpfungs-Index (%) |
|---|---|---|---|---|---|---|
| unbehandelt | - | - | 44,3 | 57,5 | 18,8 | 0 |
| A-1 | 0,25 % | M-1 | 62,3 | 38,7 | 38,4 | 24 |
| | 0,025 % | M-2 | 48,7 | 41,7 | 28,4 | 12 |
| | 0,25 g/kg Samen | M-3 | 56,0 | 39,7 | 33,8 | 18 |
| (Vergleichs-Verbindung) | | | | | | |
| 1 | 0,25 % | M-1 | 91 | 0 | 91 | 89 |
| | 0,025 % | M-2 | 89 | 1,5 | 87,7 | 85 |
| | 0,25 g/kg Samen | M-3 | 90 | 0 | 90 | 88 |
| 42 | 0,25 % | M-1 | 92 | 0 | 92 | 90 |
| | 0,025 % | M-2 | 90 | 0,5 | 89,6 | 87 |
| | 0,25 g/kg Samen | M-3 | 90 | 0 | 90 | 88 |
| 68 | 0,25 % | M-1 | 90 | 0 | 90 | 88 |
| | 0,025 % | M-2 | 90 | 0 | 90 | 88 |
| | 0,25 g/kg Samen | M-3 | 90 | 0 | 90 | 88 |

Anmerkung: Der Bekämpfungs-Index ist der Mittelwert aus drei Test-Flächen bestehend aus je 300 Samenkörnern.

Nit 184

Beispiel G

Test auf Wirksamkeit gegen die Bakanae-Krankheit der Reispflanze (Gibberella fujikuroi) bei Samen-Behandlung:

Samen (Varietät: Koshihikari), die spontan mit der Bakane-Krankheit der Reispflanze (Gibberella fujikuroi) infiziert worden waren, wurden jeweils die in der folgenden Tabelle angegebene Zeitspanne in eine Verdünnung der Test-Verbindungen mit einer vorher festgelegten, in der folgenden Tabelle aufgeführten Konzentration, die gemäß dem nachstehenden Beispiel (a) hergestellt worden war, eingetaucht. Das behandelte Saatgut wurde an der Luft getrocknet, und die behandelten Samenkörner wurden in Setzkästen eingesät und nach der üblichen Methode angezogen. Bis zu zwanzig Tage nach der Einsaat wurden die Pflänzchen periodisch herausgezogen und untersucht, und der Bekämpfungs-Index wurde nach der nachstehenden Gleichung berechnet. Die Ergebnisse sind in der folgenden Tabelle G aufgeführt.

$$\text{Bekämpfungs-Index (\%)} = \frac{\text{Prozentsatz der erkrankten Sämlinge auf der behandelten Fläche}}{\text{Prozentsatz der erkrankten Sämlinge auf der unbehandelten Fläche}} \times 100$$

Der Bekämpfungs-Index stellt einen Mittelwert aus drei Test-Flächen bestehend aus jeweils 300 Samenkörnern dar.

Tabelle G

Test auf Wirksamkeit gegen die Bakanae-Krankheit der Reispflanze (Gibberella fujikuroi) bei Samen-Behandlung:

| Verbindung Nr. | Wirkstoff-Konzentration (%) | Eintauch-Zeit (h) | Bekämpfungs-Index (%) |
|---|---|---|---|
| A-1 | 0,5 | 1/6 | 75 |
| | 0,25 | 1/6 | 30 |
| | 0,05 | 20 | 75 |
| | 0,025 | 20 | 28 |
| (Vergleichs-Verbindung) | | | |
| 1 | 0,5 | 1/6 | 100 |
| | 0,25 | 1/6 | 100 |
| | 0,125 | 1/6 | 100 |
| | 0,05 | 20 | 100 |
| | 0,025 | 20 | 100 |
| | 0,0125 | 20 | 100 |
| 42 | 0,5 | 1/6 | 100 |
| | 0,25 | 1/6 | 100 |
| | 0,125 | 1/6 | 100 |
| | 0,05 | 20 | 100 |
| | 0,025 | 20 | 100 |
| | 0,0125 | 20 | 100 |
| 68 | 0,5 | 1/6 | 100 |
| | 0,25 | 1/6 | 100 |
| | 0,125 | 1/6 | 100 |
| | 0,05 | 20 | 100 |
| | 0,025 | 20 | 100 |
| | 0,0125 | 20 | 100 |

Beispiel H

Test auf Wirksamkeit gegenüber einem die Bakanae-Krankheit der Reispflanze verursachenden, MBC-resistenten Fungus (Gibberella fujikuroi) bei Samen-Behandlung:

Der Begriff "die Bakanae-Krankheit der Reispflanze verursachender, MBC-resistenter Fungus" bezeichnet einen Pilz, der gegen Benomyl /‾ 1-(N-n-Butylcarbamoyl-2-(methoxycarboxamido)benzimidazol_7, Thiophanat-methyl /‾ 1,1-o-Phenylen-bis(3,3'-methoxycarbonyl-thioharnstoff_7 und MBC /‾oder Carbendazim, nämlich Benzimidazol-2-carbamid-säure-methylester_7 Resistenz zeigt.

Der die Bakanae-Krankheit der Reispflanze verursachende, MBC-resistente Pilz (die minimale wachstumshemmende Konzentration für Benomyl in vitro betrug 800 ppm) wurde auf Reis (Varietät: Koshihikari) im Blüh-Stadium aufgesprüht. Die erhaltenen infizierten Samen wurden durch Eintauchen in gleicher Weise wie in Beispiel C behandelt. Die Ergebnisse sind in der Tabelle H aufgeführt.

Der Bekämpfungs-Index war ein Mittelwert aus drei Test-Flächen, wobei eine Fläche jeweils aus 300 Samenkörnern bestand.

Tabelle H

Test auf Wirksamkeit gegenüber einem die Bakanae-Krankheit der Reispflanze verursachenden, MBC-resistenten Fungus (Gibberella fujikuroi) bei Samen-Behandlung:

| Verbindung Nr. | Wirkstoff-Konzentration (%) | Eintauch-Zeit (h) | Bekämpfungs-Index (%) |
|---|---|---|---|
| A-1 (Vergleichs-Verbindung) | 0,05 | 20 | 45 |
| 68 | 0,025 | 20 | 100 |

Formulierungsbeispiele:

Beispiel (a) (Benetzbares Pulver)

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Pathogene und/oder den Ort ihres Auftretens und den Ort, an dem eine Pflanzenkrankheit auftritt, aufgesprüht.

Beispiel (b) (Emulgierbares Konzentrat)

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren

miteinander vermischt, wodurch ein emulgierbares Konzentrat hergestellt wird. Dieses wird mit Wasser verdünnt und auf Pathogene und/oder den Ort ihres Auftretens und den Ort, an dem eine Pflanzenkrankheit auftritt, aufgesprüht.

Beispiel (c) (Stäubemittel)

2 Teile der Verbindung Nr. 3 der Erfindung und 98 Teile Tonpulver werden pulverisiert und gemischt, wodurch ein Stäubemittel hergestellt wird. Dieses wird über Pathogenen und/oder dem Ort ihres Auftretens und dem Ort, an dem eine Pflanzenkrankheit auftritt, ausgestreut.

Beispiel (d) (Stäubemittel)

Die Verbindung Nr. 4 der Erfindung (1,5 Teile), 0,5 Teile Isopropylhydrogenphosphat und 98 Teile Tonpulver werden pulverisiert und gemischt, wodurch ein Stäubemittel hergestellt wird. Dieses wird über Pathogenen und/oder dem Ort ihres Auftretens dem Ort, an dem eine Pflanzenkrankheit auftritt, ausgestreut.

Beispiel (e) (Granulat)

Wasser (25 Teile) wird zu einer Mischung aus 10 Teilen der Verbindung Nr. 3 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat hergestellt wird. Das Granulat wird über Pathogenen und/oder dem Ort ihres Auftretens und dem Ort, an dem eine Pflanzenkrankheit auftritt, ausgestreut.

Nit 184

## P a t e n t a n s p r ü c h e

1. Carbamoylimidazol-Derivate der allgemeinen Formel (I)

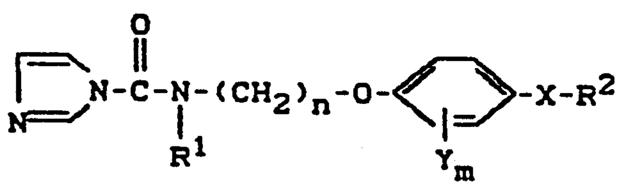

(I)

,

in der

$R^1$ eine Niederalkyl-Gruppe, eine Niederalkoxy-niederalkyl-Gruppe oder eine Cycloalkyl-Gruppe mit 3 bis 8 Kohlenstoff-Atomen bezeichnet,

$R^2$ eine fluoro-substituierte Niederalkyl-Gruppe bezeichnet,

X Sauerstoff oder Schwefel bezeichnet,

Y Halogen oder Niederalkyl bezeichnet,

m die Zahlen 0, 1 oder 2 bezeichnet und

n die Zahlen 2, 3, 4, 5 oder 6 bezeichnet.

2. Verbindungen der allgemeinen Formel (I) nach Anspruch 1, dadurch gekennzeichnet, daß

$R^1$ eine Alkyl-Gruppe mit 1 bis 4 Kohlenstoff-Atomen, eine Alkoxy-alkyl-Gruppe mit insgesamt 2 bis 4 Kohlenstoff-Atomen oder eine Cycloalkyl-Gruppe mit 5 bis 6 Kohlenstoff-Atomen bezeichnet,

$R^2$ eine Fluoroalkyl-Gruppe mit 1 bis 4 Kohlenstoff-Atomen und 1 bis 3 Fluor-Atomen bezeichnet,

Y Chlor oder Methyl bezeichnet und

X, m und n die im Vorstehenden angegebenen Bedeutungen haben.

3. Verfahren zur Herstellung von Carbamoylimidazol-Derivaten der allgemeinen Formel (I)

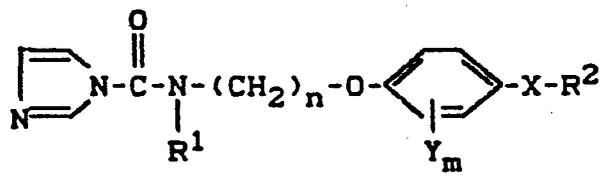

(I)

,

in der

$R^1$ eine Niederalkyl-Gruppe, eine Niederalkoxy-niederalkyl-Gruppe oder eine Cycloalkyl-Gruppe mit 3 bis 8 Kohlenstoff-Atomen bezeichnet,

$R^2$ eine fluoro-substituierte Niederalkyl-Gruppe bezeichnet,

X Sauerstoff oder Schwefel bezeichnet,

Y Halogen oder Niederalkyl bezeichnet,

m die Zahlen 0, 1 oder 2 bezeichnet und

n die Zahlen 2, 3, 4, 5 oder 6 bezeichnet,

dadurch gekennzeichnet, daß

(a) Verbindungen der Formel (II)

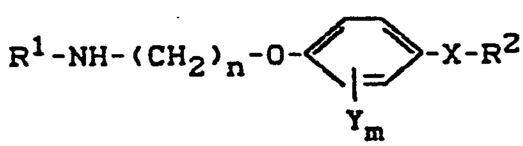

(II)

,

in der $R^1$, $R^2$, X, Y, m und n die im Vorstehenden angegebenen Bedeutungen haben, mit N,N'-Carbonyldiimidazol der Formel

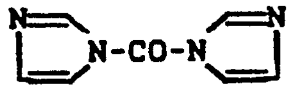

(III)

,

gegebenenfalls in Gegenwart von Säure-Acceptoren und inerten Lösungsmitteln, umgesetzt werden oder

(b) Verbindungen der Formel (IV)

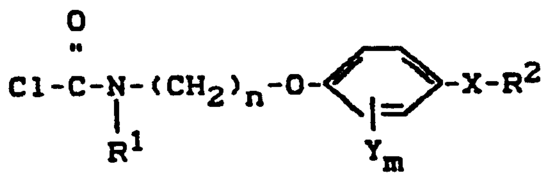

(IV)

,

in der $R^1$, $R^2$, X, Y, m und n die im Vorstehenden angegebenen Bedeutungen haben, mit der Imidazol-Verbindung der Formel (V)

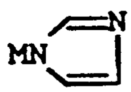

(V)

,

in der M ein Wasserstoff-Atom oder ein Alkalimetall-Atom bezeichnet, gegebenenfalls in Gegenwart von Säure-Acceptoren und inerten Lösungsmitteln umgesetzt werden.

4. Fungizide Zusammensetzung für Landwirtschaft und Gartenbau, dadurch gekennzeichnet, daß sie wenigstens ein Carbamoylimidazol-Derivat der Formel (I) enthält.

5. Verwendung von Carbamoylimidazol-Derivaten der Formel (I) zur Verhütung von Pflanzenkrankheiten.

6. Verfahren zur Bekämpfung von Pflanzenkrankheiten, dadurch gekennzeichnet, daß ein Carbamoylimidazol-Derivat der Formel (I), entweder allein oder im Gemisch mit einem Verdünnungsmittel und oder einem grenzflächenaktiven Mittel und, falls weiter erforderlich, mit einem Stabilisator, einem Haftmittel oder einem synergistischen Mittel zur Einwirkung gebracht wird.

7. Verbindungen der Formel (VI)

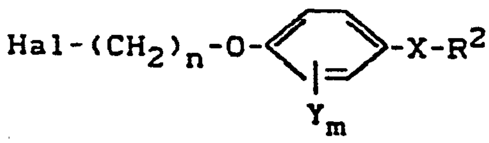

(VI)

,

in der

$R^2$ eine fluoro-substituierte Niederalkyl-Gruppe bezeichnet,

X Sauerstoff oder Schwefel bezeichnet,

Y Halogen oder Niederalkyl bezeichnet,

m die Zahlen 0, 1 oder 2 bezeichnet und

n die Zahlen 2, 3, 4, 5 oder 6 bezeichnet.

8. Verfahren zur Herstellung von Verbindungen der Formel (VI)

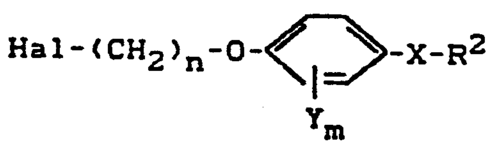

(VI)

,

in der

$R^2$ eine fluoro-substituierte Niederalkyl-Gruppe bezeichnet,

X Sauerstoff oder Schwefel bezeichnet,
Y Halogen oder Niederalkyl bezeichnet,
m die Zahlen 0, 1 oder 2 bezeichnet und
n die Zahlen 2, 3, 4, 5 oder 6 bezeichnet,

dadurch gekennzeichnet, daß Verbindungen der Formel (VIII)

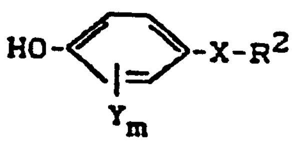

(VIII)

,

in der
$R^2$, X, Y, und m die im Vorstehenden angegebenen Bedeutungen haben,
mit Dihalogeno-Verbindungen der Formel (IX)

$$Hal-(CH_2)_n-Hal \qquad (IX),$$

in der
n die im Vorstehenden angegebene Bedeutung hat und
Hal Halogen bezeichnet,

gegebenenfalls in Gegenwart von Säure-Acceptoren und inerten Lösungsmitteln, umgesetzt werden.

9. Verfahren zur Herstellung von Verbindungen der Formel (II)

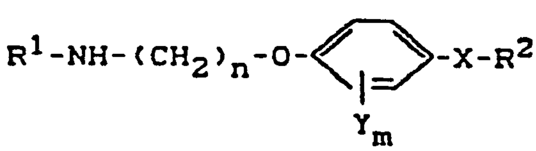

(II)

,

in der
$R^1$ eine Niederalkyl-Gruppe, Niederalkoxy-niederalkyl-Gruppe oder Cycloalkyl-Gruppe mit 3 bis 8 Kohlenstoff-Atomen bezeichnet,

$R^2$ eine fluoro-substituierte Niederalkyl-Gruppe bezeichnet,

X Sauerstoff oder Schwefel bezeichnet,

Y Halogen oder Niederalkyl bezeichnet,

m die Zahlen 0, 1 oder 2 bezeichnet und

n die Zahlen 2, 3, 4, 5 oder 6 bezeichnet,

dadurch gekennzeichnet, daß die Verbindungen der Formel (VI)

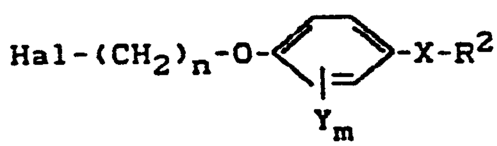

(VI)

,

in der

$R^2$, X, Y, m und n die im Vorstehenden angegebenen Bedeutungen haben,

mit Amino-Verbindungen der Formel (VII)

$$R^1\text{-}NH_2 \qquad (VII),$$

in der

$R^1$ die im Vorstehenden angegebene Bedeutung hat,

umgesetzt werden.

10. Verfahren zur Herstellung von Verbindungen der Formel (IV)

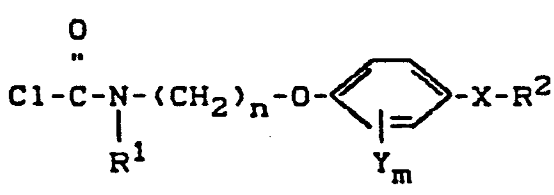

(IV)

,

in der

$R^1$ eine Niederalkyl-Gruppe, Niederalkoxy-niederalkyl-Gruppe oder Cycloalkyl-Gruppe mit 3 bis 8 Kohlenstoff-Atomen bezeichnet,

$R^2$ eine fluoro-substituierte Niederalkyl-Gruppe bezeichnet,

X Sauerstoff oder Schwefel bezeichnet,

Y Halogen oder Niederalkyl bezeichnet,

m die Zahlen 0, 1 oder 2 bezeichnet und

n die Zahlen 2, 3, 4, 5 oder 6 bezeichnet,

dadurch gekennzeichnet, daß die Verbindungen der Formel (II)

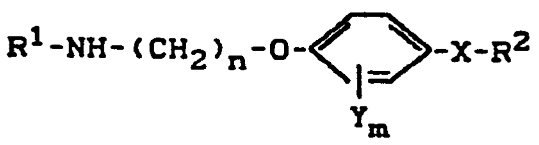

(II)

,

in der

$R^1$, $R^2$, X, Y, m und n die im Vorstehenden angegebenen Bedeutungen haben,

mit Phosgen, vorzugsweise in einem Lösungsmittel, umgesetzt werden.

0175188

Nummer der Anmeldung

Europäisches Patentamt

# EUROPÄISCHER RECHERCHENBERICHT

EP 85110918.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,A | GB - A - 1 469 772 (THE BOOTS COMPANY LTD.)<br>* Ansprüche 1,2,15-18; Seite 5, Zeilen 53-59 *<br>-- | 1,3,4-6,10 | C 07 D 233/61<br>C 07 C 43/225<br>C 07 C 149/34<br>C 07 C 149/42<br>C 07 C 149/437<br>A 01 N 47/38 |
| A | FR - A - 2 499 077 (CIBA-GEIGY AG)<br>* Ansprüche 1, 9-12 *<br>-- | 1,3,4-6 | |
| A | GB - A - 2 058 061 (EGYT GYOGYSZERVEGYESZETI GYAR)<br>* Anspruch 1; Abstract *<br>-- | 7,8 | |
| A | DE - A1 - 2 930 608 (BRISTOL-MYERS CO)<br>* Ansprüche 1,6 *<br>-- | 9 | RECHERCHIERTE SACHGEBIETE (Int. Cl.4) |
| A | GB - A - 2 097 000 (RIKER LABORATORIES INC.)<br>* Abstract *<br>-- | 7 | C 07 D 233/00<br>C 07 C 43/00<br>C 07 C 149/00 |
| A | EP - A2 - 0 032 063 (WARNER-LAMBERT COMPANY)<br>* Anspruch 1 *<br>---- | 7 | |

Der vorliegende Recherchenbericht wurde für alle Patentansprüche erstellt.

| Recherchenort | Abschlußdatum der Recherche | Prüfer |
|---|---|---|
| WIEN | 11-12-1985 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82